# EUROPEAN PATENT APPLICATION

(11) **EP 4 810 529 A1**
(43) Date of publication of application: **23.09.2026**
(21) Application number: 24891353.5
(22) Date of filing: 11.11.2024
(51) Int. Cl.: C08F 2/40, B32B 27/00, C07C 255/09, C07C 309/04, C08F 20/42, C08F 22/34, C08F 222/32

(54) **1,1-DICYANOETHYLENE-CONTAINING COMPOSITION, CURED PRODUCT, LAMINATE, AND METHOD FOR PRODUCING 1,1-DICYANOETHYLENE-CONTAINING COMPOSITION**

(30) Priority: 13.11.2023 JP 2023193003; 28.03.2024 JP 2024052789
(71) Applicant: Kuraray Co., Ltd., Kurashiki-shi, Okayama 710-0801 (JP)
(72) Inventor: INATOMI Atsushi, Tsukuba-shi, Ibaraki 305-0841 (JP); SHIROTA Kentaro, Tsukuba-shi, Ibaraki 305-0841 (JP)
(74) Representative: Müller-Boré & Partner Patentanwälte PartG mbB
(86) International application number: PCT/JP2024/039899
(87) International publication number: WO 2025/105317

(57) **Abstract**

Provided is a 1,1-dicyanoethylene-containing composition containing 1,1-dicyanoethylene, water, and an acidic compound (A), in which a content of the acidic compound (A) and a content of the water in the composition satisfy a specific relational expression (Ia): -4.00 ≤ α ≤ 0, in which α is represented by α = pKa + Log(C_{H2O}/Cₐ), pKa is a logarithmic acid dissociation constant of the acidic compound (A), C_{H2O} is a molar concentration of the water in the composition, and Cₐ is a molar concentration of the acidic compound (A) in the composition.

## Description

### Technical Field

The present invention relates to a 1,1-dicyanoethylene-containing composition, a cured product, a laminate, and a method for producing a 1,1-dicyanoethylene-containing composition.

### Background Art

Since 1,1-dicyanoethylene is excellent in reactivity, it is sometimes used for a curable adhesive.

However, due to its high reactivity, it is essential to use an acid stabilizer to enhance the storage stability in order to use 1,1-dicyanoethylene. PTLs 1 and 2 propose the use of benzenesulfonic acid, chlorobenzenesulfonic acid, p-toluenesulfonic acid, or the like as an acid stabilizer.

Methylenemalonic acid is a compound having high reactivity similar to 1,1-dicyanoethylene. PTL 3 proposes to enhance storage stability by allowing an antioxidant and an acid to coexist with methylenemalonic acid. In PTL 3, specifically, acetic acid is used as an acid in Examples.

### Citation List

### Patent Literature

PTL 1: US 2,665,298
PTL 2: US 2,535,861
PTL 3: JP 2008-174494 A

### Summary of Invention

### Technical Problem

However, as a result of studies by the present inventors, it has been found that in the methods described in PTLs 1, 2, and 3, stability is deteriorated, for example, solids are generated due to an excessively high acid concentration. In addition, as a result of studies by the present inventors, it has been found that the polymerization of 1,1-dicyanoethylene cannot be sufficiently suppressed in some cases even when acetic acid described in PTL 3 is used.

Thus, there is still a need to improve the storage stability of a 1,1-dicyanoethylene-containing composition and to provide a method for producing a 1,1-dicyanoethylene-containing composition having excellent storage stability. In addition, if a 1,1-dicyanoethylene-containing composition having excellent storage stability can be provided, it is expected that a cured product and a laminate that do not impair the expected physical properties can be provided.

An object of the present invention is to provide a 1,1-dicyanoethylene-containing composition having excellent storage stability and a method for producing the same. Another object of the present invention is to provide a cured product using the same components as those of the 1,1-dicyanoethylene-containing composition, and a laminate containing the cured product.

### Solution to Problem

The present inventors have conducted intensive studies on the reason why polymerization of 1,1-dicyanoethylene cannot be sufficiently suppressed only by adding an arbitrary acidic compound to 1,1-dicyanoethylene, and have found that the amount of water and the amount of acid in a 1,1-dicyanoethylene-containing composition have an influence on the suppression. Further, the present inventors have found that by blending water and an acid such that the molar concentration of water and the molar concentration of the acid satisfy a specific relational expression, it is possible to obtain a 1,1-dicyanoethylene-containing composition having excellent storage stability, thereby completing the present invention.

Specifically, the present invention provides the following [1] to [11].
[1] A 1,1-dicyanoethylene-containing composition containing 1,1-dicyanoethylene, water, and an acidic compound (A), in which a content of the acidic compound (A) and a content of the water in the composition satisfy the following relational expression (Ia): $- 4.00 \leq α \leq 0$ in the relational expression (Ia), α is represented by the following formula (Ib): $α = pKa + Log \left({C}_{H 2 O}/{C}_{a}\right)$ in the formula (Ib), pKa is a logarithmic acid dissociation constant of the acidic compound (A); C_{H2O} is a molar concentration of the water in the composition; and Cₐ is a molar concentration of the acidic compound (A) in the composition.
[2] The 1,1-dicyanoethylene-containing composition according to [1], in which the acidic compound (A) is selected from acidic compounds having a logarithmic acid dissociation constant pKa of -1.7 or less.
[3] The 1,1-dicyanoethylene-containing composition according to [1] or [2], in which the acidic compound (A) is at least one selected from methanesulfonic acid, sulfuric acid, p-toluenesulfonic acid, and hydrochloric acid.
[4] The 1,1-dicyanoethylene-containing composition according to any one of [1] to [3], further containing a polymerizable monomer (B).
[5] The 1,1-dicyanoethylene-containing composition according to [4], in which the polymerizable monomer (B) is one or more selected from the group consisting of ethylene, propylene, butadiene, isobutylene, isoprene, 1-hexene, 1-octene, vinyl acetate, vinyl propionate, vinyl butyrate, styrene, α-methylstyrene, p-methylstyrene, acrylic acid, methacrylic acid, alkyl acrylate, alkyl methacrylate, acrylonitrile, vinyl chloride, vinylidene chloride, vinylidene fluoride, 2-cyanoacrylic acid alkyl ester, 2-cyanopentadienoic acid alkyl ester, and methylidenemalonic acid dialkyl ester.
[6] The 1,1-dicyanoethylene-containing composition according to [5], in which the polymerizable monomer (B) contains 2-cyanoacrylic acid alkyl ester.
[7] The 1,1-dicyanoethylene-containing composition according to [6], in which the 2-cyanoacrylic acid alkyl ester is ethyl 2-cyanoacrylate.
[8] The 1,1-dicyanoethylene-containing composition according to any one of [1] to [7], in which the molar concentration of the acidic compound (A) in the composition is 1,000 mmol/L or less.
[9] A cured product obtained by reacting the 1,1-dicyanoethylene-containing composition according to any one of [1] to [8].
[10] A laminate containing: a cured product obtained by reacting the 1,1-dicyanoethylene-containing composition according to any one of [1] to [8]; and an adherend bonded to the cured product.
[11] A method for producing a 1,1-dicyanoethylene-containing composition, the method including a step of blending an acidic compound (A) such that a content of the acidic compound (A) in the composition and a content of water in the composition satisfy the following relational expression (Ia): $- 4.00 \leq α \leq 0$ in the relational expression (Ia), α is represented by the following formula (Ib): $α = pKa + Log \left({C}_{H 2 O}/{C}_{a}\right)$ in the formula (Ib), pKa is a logarithmic acid dissociation constant of the acidic compound (A); C_{H2O} is a molar concentration of the water in the composition; and Cₐ is a molar concentration of the acidic compound (A) in the composition.

### Advantageous Effects of Invention

According to the present invention, it is possible to provide a 1,1-dicyanoethylene-containing composition having excellent storage stability and a method for producing the same. In addition, according to the present invention, it is possible to provide a cured product using the same components as those of the 1,1-dicyanoethylene-containing composition, and a laminate containing the cured product.

### Description of Embodiments

Hereinafter, a description will be given based on an example of a mode for carrying out the present invention (hereinafter, sometimes referred to as "the present embodiment"). However, the embodiments described below are illustrative for embodying the technological thought of the present invention, and the present invention is not limited to the following descriptions.

In addition, in the description herein, preferred modes of the embodiments are shown, but a combination of two or more of individual preferred modes is also a preferred mode. Regarding the matters indicated by numerical ranges, in a case where there are several numerical ranges, it is possible to selectively combine a lower limit value and an upper limit value thereof to obtain a preferred mode.

In the description herein, when there is a description pertaining to a numerical range of "XX to YY", the description means "XX or more and YY or less".

### [1,1-Dicyanoethylene-Containing Composition]

A 1,1-dicyanoethylene-containing composition according to an embodiment of the present invention is a 1,1-dicyanoethylene-containing composition containing 1,1-dicyanoethylene, water, and an acidic compound (A), in which a content of the acidic compound (A) and a content of the water in the composition satisfy the following specific relational expression (Ia): $- 4.00 \leq α \leq 0$ in the relational expression (Ia), α is represented by the following formula (Ib): $α = pKa + Log \left({C}_{H 2 O}/{C}_{a}\right)$ in the formula (Ib), pKa is a logarithmic acid dissociation constant of the acidic compound (A); C_{H2O} is a molar concentration of the water in the composition; and Cₐ is a molar concentration of the acidic compound (A) in the composition.

In the 1,1-dicyanoethylene-containing composition, the water content and the content of the acidic compound (A) satisfy a specific relational expression (Ia), whereby it is possible to provide a 1,1-dicyanoethylene-containing composition having excellent storage stability. The acidic compound (A) may be one kind or a plurality of kinds.

### (1,1-Dicyanoethylene)

1,1-Dicyanoethylene may be produced according to the production method described in J. Am. Chem. Soc., 1989, 111, 9078-9081 or the production method described in U.S. Patent No. 2476270. 1,1-Dicyanoethylene is preferably produced by the production method described in Production Example 1 described later. The produced 1,1-dicyanoethylene is preferably stored in a frozen state or in the state of coexisting with the acidic compound (A) until immediately before use.

In addition, the produced 1,1-dicyanoethylene may be stored in the state of coexisting with, for example, an aromatic solvent such as toluene or xylene; an aliphatic solvent such as hexane or heptane; a naphthene solvent such as cyclohexane; an ester solvent such as ethyl acetate; or an ether solvent such as tetrahydrofuran or diethyl ether until immediately before use.

### (Purity)

The purity of 1,1-dicyanoethylene to be used in the 1,1-dicyanoethylene-containing composition according to the embodiment of the present invention is preferably 95% or more, more preferably 97% or more, still more preferably 98% or more, and yet still more preferably 99% or more. The purity of 1,1-dicyanoethylene can be determined by, for example, gas chromatography.

### (Water Content of 1,1-Dicyanoethylene)

The 1,1-dicyanoethylene may contain a trace amount of water depending on the production method or purification method thereof. The trace amount of water is preferably small, but is, for example, 10,000 mmol/L or less, preferably 1,000 mmol/L or less, more preferably 100 mmol/L or less, still more preferably 50 mmol/L or less, and yet still more preferably 25 mmol/L or less, with respect to the total amount of 1,1-dicyanoethylene. The water content of 1,1-dicyanoethylene can be quantified as the water content of the composition in the presence of the acidic compound (A).

### (Water)

The 1,1-dicyanoethylene-containing composition according to the present embodiment contains water. This amount of water is the total amount of the water content of 1,1-dicyanoethylene, the water content of the acidic compound (A), and the amount of water to be added. Note that water can be added, for example, for illustration in Examples or Comparative Examples described later. In addition, water can be added so as to satisfy the above-described relational expression even when the acidic compound (A) is added in excess. The amount (total amount) of water in the 1,1-dicyanoethylene-containing composition can be quantified by a method (specifically, the Karl Fischer titration method) described in Examples described later.

### (Acidic Compound (A))

The acidic compound (A) according to the present embodiment is not limited as long as it satisfies the above-described specific relational expression (Ia). Preferably, the acidic compound (A) is selected, and the amount of the selected acidic compound is determined so as to satisfy the relational expression (Ia).

From the viewpoint of satisfying the relational expression (Ia), the acidic compound (A) is preferably selected from acidic compounds having a logarithmic acid dissociation constant pKa of -1.7 or less. The logarithmic acid dissociation constant pKa of the acidic compound (A) is usually a logarithmic acid dissociation constant at room temperature in an aqueous system. For the logarithmic acid dissociation constant, reference may be made to Non-Patent Literature 1 (pKa table by D. H. Ripin, D. A. Evans; available from https://depts.washington.edu/eooptic/linkfiles/evans_pKa_table.pdf). When the acidic compound (A) is dissociated in multiple stages, the logarithmic acid dissociation constant pKa is usually the logarithmic acid dissociation constant of the first stage. The logarithmic acid dissociation constant of the acidic compound (A) not described in Non-Patent Literature 1 may be determined by a method known to those skilled in the art (for example, a neutralization titration method). Although not disclosed in Non-Patent Literature 1, the pKa of p-toluenesulfonic acid monohydrate is -1.7.

The acidic compound (A) having a logarithmic acid dissociation constant pKa of -1.7 or less can efficiently protonate water (H₂O) in the 1,1-dicyanoethylene-containing composition to form oxonium ions (H₃O⁺). From such a viewpoint, the logarithmic acid dissociation constant pKa of the acidic compound (A) is more preferably -1.8 or less, still more preferably -1.9 or less, yet still more preferably -2.0 or less, and even more preferably -2.1 or less.

Specific examples of the acidic compound (A) include methanesulfonic acid (pKa: -2.6), sulfuric acid (pKa: -3.0), hydrochloric acid (pKa: -8.0), nitric acid (pKa: -1.3), sulfurous acid (pKa: 1.9), and trifluoroacetic acid (pKa: -0.25). In one aspect of the present embodiment, the acidic compound (A) is at least one selected from methanesulfonic acid, sulfuric acid, trifluoroacetic acid, hydrochloric acid, and nitric acid, and preferably at least one selected from methanesulfonic acid, sulfuric acid, and hydrochloric acid. In another aspect of the present embodiment, the acidic compound (A) is at least one selected from methanesulfonic acid, sulfuric acid, p-toluenesulfonic acid, and hydrochloric acid.

### (Specific Relational Expression)

In the present embodiment, the above-described relational expression is shown again as follows. $- 4.00 \leq α \leq 0$ in the relational expression (Ia), α is represented by the following formula (Ib): $α = {pK}_{a} + Log \left({C}_{H 2 O}/{C}_{a}\right)$ in the formula (Ib), pKa is a logarithmic acid dissociation constant of the acidic compound (A); C_{H2O} is a molar concentration of the water in the composition; and Cₐ is a molar concentration of the acidic compound (A) in the composition.

In the above relational expression (Ia), α is preferably -3.90 or more, more preferably -3.80 or more, still more preferably -3.70 or more, and yet still more preferably -3.60 or more from the viewpoint of reliably achieving the expected effect of the present invention.

Incidentally, in a case where two kinds of acidic compounds (A) are used, it is preferable that the relational expression (Ib) is regarded as the following formula (Ib-2). ${10}^{α} = \left\{{C}_{a 1}\times {K}_{a 1}/\left({K}_{a 1}+{10}^{α}\right)\right\} + \left\{{C}_{a 2}\times {K}_{a 2}/\left({K}_{a 2}+{10}^{α}\right)\right\}$

In the formula (Ib-2), Cₐ₁ is a molar concentration of the first kind of acidic compound, Kₐ₁ is an acid dissociation constant of the first kind of acidic compound, Cₐ₂ is a molar concentration of the second kind of acidic compound, and Kₐ₂ is an acid dissociation constant of the second kind of acidic compound.

In a case where there are three or more kinds of acidic compounds (A), the sum on the right side may be calculated in consideration of the molar concentration and the acid dissociation constant of each acidic compound in the same manner as in the formula (Ib-2).

In the present embodiment, the reason why the 1,1-dicyanoethylene-containing composition having excellent storage stability is obtained by the content of the acidic compound (A) and the content of water satisfying a specific relational expression (Ia) is not certain, but the present inventors assume as follows.

1,1-Dicyanoethylene is stabilized in the composition by the formation of oxonium ions by the water contained in the 1,1-dicyanoethylene-containing composition. As a result, it is considered that the polymerization of 1,1-dicyanoethylene is inhibited, and an increase in viscosity or the formation of a precipitate (these phenomena are exemplified and confirmed in the section of Examples) is suppressed. Here, the ability to form oxonium ions is possessed by the acidic compound (A) in the 1,1-dicyanoethylene-containing composition. Therefore, the content of water and the acidic compound (A) have a close relationship. This has been clarified for the first time by the examples in the section of Examples described later. As the content of water increases, water can be efficiently converted into oxonium ions by increasing the content of the acidic compound (A), by selecting an acidic compound having excellent ability to form oxonium ions, for example, an acidic compound having a smaller pKa value as described above, as the acidic compound (A), or by satisfying both conditions. On the other hand, when the content of the acidic compound (A) is too large, a colored precipitate derived from 1,1-dicyanoethylene in the 1,1-dicyanoethylene-containing composition tends to be generated. It is considered that this is because a side reaction in which the acidic compound (A) reacts with a cyano group occurs. Since the pKa value of a strongly acidic compound such as sulfuric acid decreases (the absolute value of the pKa value increases) as the amount of water in the composition decreases, it is considered that the reactivity with the cyano group is improved as a result of the small amount of water with respect to the acid, and the side reaction is promoted. From the above, it can be said that the acidic compound (A) is required to have a pKa value that is low to some extent, and is required to be in an amount corresponding to the content of water, that is, an amount sufficient for converting water into oxonium ions, and not to be in an excessive amount from the viewpoint of suppressing coloring. However, since the amount of the precipitate can be minimized as long as 1,1-dicyanoethylene can be stabilized, the problem of color suppression can be regarded as a secondary problem. As a result of such studies, in the present embodiment, the pKa and the molar concentration of the acidic compound (A) are used to express the above-mentioned relational expression.

### (Content of Acidic Compound (A))

The content of the acidic compound (A) is not limited as long as it satisfies the above-mentioned relational expression. The mass ratio of the content of the acidic compound (A) to the content of water is, for example, in the range of 1:0.001 to 0.0001:1, preferably in the range of 1:0.01 to 0.01:1, more preferably in the range of 1:0.1 to 0.1:1, and still more preferably in the range of 1:0.1 to 0.1:0.6. From the viewpoint of satisfying the above relational expression and converting as much water as possible into oxonium ions, the content of the acidic compound (A) is preferably the same as or larger than the content of water (that is, the value of (C_{H2O}/Cₐ) in the relational expression (Ib) is 1 or less).

The molar concentration of the acidic compound (A) in the 1,1-dicyanoethylene-containing composition is not limited as long as the above relational expression is satisfied. From the viewpoint of suppressing coloration of the 1,1-dicyanoethylene-containing composition, the molar concentration of the acidic compound (A) is preferably 1,000 mmol/L or less, more preferably 500 mmol/L or less, still more preferably 150 mmol/L or less, and yet still more preferably 20 mmol/L or less in the 1,1-dicyanoethylene-containing composition. On the other hand, from the viewpoint of the storage stability of the 1,1-dicyanoethylene-containing composition, the molar concentration of the acidic compound (A) is preferably 0.001 mmol/L or more in the 1,1-dicyanoethylene-containing composition.

### (Water Content of Acidic Compound (A))

The acidic compound (A) may contain a trace amount of water depending on the production method or purification method thereof. The trace amount of water can be quantified as the water content of the composition in the presence of 1,1-dicyanoethylene.

### (Component (B): Polymerizable Monomer)

The 1,1-dicyanoethylene-containing composition according to the present embodiment may further contain a polymerizable monomer (B). The polymerizable monomer (B) excludes 1,1-dicyanoethylene. The polymerizable monomer (B) is preferably reactive with 1,1-dicyanoethylene. The polymerizable monomer (B) may be one kind or a plurality of kinds. The polymerizable monomer (B) may be a radically polymerizable monomer or an anionically polymerizable monomer.

Examples of the polymerizable monomer (B) include ethylene, propylene, butadiene, isobutylene, isoprene, 1-hexene, 1-octene, vinyl acetate, vinyl propionate, vinyl butyrate, styrene, α-methylstyrene, p-methylstyrene, acrylic acid, methacrylic acid, alkyl acrylate esters such as butyl acrylate, alkyl methacrylate esters such as methyl methacrylate and dodecyl methacrylate, acrylonitrile, vinyl chloride, vinylidene chloride, vinylidene fluoride, 2-cyanoacrylic acid alkyl ester, 2-cyanopentadienoic acid alkyl ester, and methylidenemalonic acid dialkyl ester. Among these, from the viewpoint of excellent reactivity with 1,1-dicyanoethylene, the polymerizable monomer (B) is preferably a 2-cyanoacrylic acid alkyl ester, and the 2-cyanoacrylic acid alkyl ester is more preferably ethyl 2-cyanoacrylate.

The method for producing the polymerizable monomer (B) is not particularly limited, and the polymerizable monomer (B) can be produced by a known method alone or in combination. The polymerizable monomer (B) may be a commercially available product.

### (Other Component)

The 1,1-dicyanoethylene-containing composition according to the present embodiment may contain one or more other components.

Examples of such other components include a thickener, a dehydrating agent, a radical polymerization inhibitor, a plasticizer, a pigment, an organic solvent, rubber, an organic filler, and an inorganic filler. The other components can be used in such an amount that the expected effect of the present invention is not impaired. The rubber may also function as an organic filler. The inorganic filler may also function as a thickener.

From the viewpoint of heat resistance and moisture resistance, the 1,1-dicyanoethylene-containing composition according to the present embodiment preferably does not contain ethyl 2-cyanoacrylate, butyl 2-cyanoacrylate, and diethyl methylidenemalonate.

### (Content of Each Component)

The total amount of the monomers (1,1-dicyanoethylene and the optional polymerizable monomer (B) used as needed) is preferably 70% by mass or more, more preferably 80% by mass or more, and still more preferably 90% by mass or more, based on 100% by mass of the entire 1,1-dicyanoethylene-containing composition according to the present embodiment, from the viewpoint of achieving the expected physical properties. When the polymerizable monomer (B) is used, the mass ratio of 1,1-dicyanoethylene to the polymerizable monomer (B) is not particularly limited, and may be 99:1 to 1:99, 80:20 to 20:80, or 70:30 to 30:70.

### [Method for Producing 1,1-Dicyanoethylene-Containing Composition]

A method for producing a 1,1-dicyanoethylene-containing composition according to an embodiment of the present invention is a method for producing a 1,1-dicyanoethylene-containing composition, the method including a step (blending step) of blending an acidic compound (A) such that a content of the acidic compound (A) in the composition and a content of water in the composition satisfy the following relational expression (Ia): $- 4.00 \leq α \leq 0$ in the relational expression (Ia), α is represented by the following formula (Ib): $α = pKa + Log \left({C}_{H 2 O}/{C}_{a}\right)$ in the formula (Ib), pKa is a logarithmic acid dissociation constant of the acidic compound (A); C_{H2O} is a molar concentration of the water in the composition; and Cₐ is a molar concentration of the acidic compound (A) in the composition.

By employing the above production method, it is possible to produce the 1,1-dicyanoethylene-containing composition according to the above embodiment, that is, a 1,1-dicyanoethylene-containing composition having excellent storage stability. In the blending step, stirring may or may not be performed. In addition, in the blending step, when the water content is known, the type and the blending amount of the acidic compound (A) are preferably determined so as to satisfy the above relational expression. When the blending amount of the acidic compound (A) is excessive (or has to be excessive) from the viewpoint of the above relational expression, water is blended so as to satisfy the above relational expression. That is, the blending step may be a step of blending water and the acidic compound (A) so that the content of the acidic compound (A) and the content of the water in the composition satisfy the above relational expression (Ia).

### (Application)

The 1,1-dicyanoethylene-containing composition according to the present embodiment can be used as an (instant) adhesive. Such adhesive applications include general household adhesive, medical applications, hemostatic adhesive, laminations, bookbinding, shoe assembly, automotive parts, air conditioners, electrical or electronic equipment components or other durable goods, assembly of components used in the building industry (e.g., insulating applications, thermo- and/or acoustical applications), packaging, die attach applications, wound closure, surgical sutures, medical device applications and labelling of all kinds, lash extension adhesive, and cosmetic adhesive.

An instant adhesive containing ethyl 2-cyanoacrylate as a main raw material is widely known, but ethyl 2-cyanoacrylate has a disadvantage of being inferior in heat resistance and moisture resistance, and the instant adhesive is inferior in heat resistance and moisture resistance. On the other hand, 1,1-dicyanoethylene has excellent heat resistance and moisture resistance, and an adhesive using the 1,1-dicyanoethylene-containing composition according to the present embodiment has excellent heat resistance and moisture resistance.

The 1,1-dicyanoethylene-containing composition according to the present embodiment can also be used as a coating material. Examples of the application of such a coating material include a film capacitor, an insulating layer of an EL element, an electrostatic induction conversion element, a sensor (for example, a touch sensor, a vibration sensor, a biosensor, and a tire sensor (particularly, a sensor installed on a tire inner surface)), an actuator, a touch panel, a haptic device, a vibration power generation device (for example, a vibration power generation floor and a vibration power generation tire), a speaker, a microphone, a vibration damping sheet, a hollow fiber membrane for water purification, and a resist film. An example of the haptic device is a device having a function of feeding back a tactile sense to a user.

### [Cured Product]

A cured product according to an embodiment of the present invention is a cured product obtained by reacting the 1,1-dicyanoethylene-containing composition described above. Specifically, the cured product according to the embodiment of the present invention is a cured product obtained by reacting a composition containing 1,1-dicyanoethylene, water, and an acidic compound (A), or a composition containing 1,1-dicyanoethylene, water, an acidic compound (A), and a polymerizable monomer (B), in which a content of the acidic compound and a content of the water in the composition satisfy the following relational expression (Ia): $- 4.00 \leq α \leq 0$ in the relational expression (Ia), α is represented by the following formula (Ib): $α = {pK}_{1} + Log \left({C}_{H 2 O}/{C}_{a}\right)$ in the formula (Ib), pK₁ is a logarithmic acid dissociation constant of the acidic compound (A); C_{H2O} is a molar concentration of the water in the composition; and Cₐ is a molar concentration of the acidic compound (A) in the composition.

### (Monomer Mixture)

1,1-dicyanoethylene may or may not constitute a monomer mixture together with the polymerizable monomer (B). In the case of a monomer mixture, 1,1-dicyanoethylene and the polymerizable monomer (B) are respectively the same as 1,1-dicyanoethylene and the polymerizable monomer (B) that can be contained in the 1,1-dicyanoethylene-containing composition according to the present embodiment described above. The monomer mixture may contain one or more other components. Such other components are also the same as the other components that can be contained in the 1,1-dicyanoethylene-containing composition described above.

### (Lewis Basic Compound)

In addition to water, a Lewis basic compound other than water may or may not be used. The Lewis basic compound usually functions as a polymerization catalyst for 1,1-dicyanoethylene or the monomer mixture. Examples of the Lewis basic compound include alcohols and alkylamines.

Examples of the alcohol include methanol, ethanol, and propanol.

Examples of the alkylamine include tertiary amines such as trimethylamine, triethylamine, tripropylamine, triisopropylamine, N,N-dimethylethylamine, N,N-dimethylpropylamine, and N,N-dimethylisopropylamine.

### (Other Additives)

The cured product according to the present embodiment may contain, as other additives, at least one selected from the group consisting of thickeners (e.g., organic thickeners, inorganic thickeners such as silica), dehydrating agents (e.g., carboxylic acid anhydrides such as acetic anhydride, cyclic sulfonic acid esters such as propane sultone, and phosphoric acid anhydrides such as diphosphorus pentoxide), radical polymerization inhibitors (e.g., phenol-based compounds, quinone-based compounds, stable radical compounds, and metal salts), plasticizers (e.g., ester compounds such as phthalic acid esters and adipic acid esters), rubbers (e.g., natural rubbers, styrene-butadiene rubbers, hydrogenated styrene-butadiene rubbers, acrylic rubbers, nitrile rubbers, and hydrogenated nitrile rubbers), pigments, and fillers (e.g., inorganic fillers and organic fillers) in an amount that does not excessively impair the expected effect of the present invention. Specific examples of the organic thickener include polymer compounds such as an ethylene-vinyl acetate copolymer, a methyl (meth)acrylate resin, a polystyrene resin, a (modified) cellulose resin, and an acrylonitrile resin. Specific examples of the phenol-based compound include BHT (dibutylhydroxytoluene). Examples of the quinone-based compound include hydroquinone. Specific examples of the stable radical compound include DPPH (2,2-diphenyl-1-picrylhydrazyl) and TEMPO (2,2,6,6-tetramethylpiperidine 1-oxyl). The inorganic thickener may also function as an inorganic filler. The above-mentioned phenolic compound is excluded from the radical polymerization inhibitor. The rubber may also function as an organic filler. Further, a hardening accelerator such as a polyethylene glycol derivative, crown ether, and calixarene can be added for the purpose of improving the bonding speed. Furthermore, a filler, an elastomer, a thixotropy imparting agent, an adhesion imparting agent, a crosslinking agent, a fragrance, and the like may be added according to the purpose.

### (Production Method of Cured Product)

The production method of the cured product is not particularly limited. For example, a cured product can be obtained by mixing the monomer mixture with the Lewis basic compound at room temperature (23°C).

The amount of the Lewis basic compound is not particularly limited, and is preferably 0.001 to 1.0 parts by mass, and more preferably 0.01 to 0.5 parts by mass with respect to 100 parts by mass of the monomer mixture. When the content of the Lewis basic compound is within the above range, the monomer mixture reacts rapidly.

### (Application)

The cured product according to the present embodiment may be a cured product of an (instant) adhesive. The use of the adhesive is as described above.

The cured product according to the present embodiment may be a coating material. The use of the coating material is as described above.

### [Laminate]

A laminate according to an embodiment of the present invention is a laminate containing a cured product obtained by reacting the 1,1-dicyanoethylene-containing composition described above and an adherend bonded to the cured product. The laminate according to the embodiment of the present invention may be a laminate containing the cured product according to the embodiment described above and an adherend bonded to the cured product. The laminated structure is not particularly limited, and the cured product may be formed on one adherend, or the cured product may be provided between two adherends. By having the cured product between two adherends, the adherends can be strongly bonded to each other.

The adherend used in the laminate of the present invention is not particularly limited, and examples thereof include synthetic resins, metals, ceramics, and fabrics.

Examples of the synthetic resin include polyolefin resins such as polyethylene, polypropylene, copolymers of ethylene and one or more α-olefins having 3 to 20 carbon atoms (for example, propylene, 1-butene, 1-pentene, and 1-hexene), an ethylene-propylene-diene copolymer (EPDM), an ethylene-vinyl acetate copolymer, and an ethylene-acrylic acid copolymer, a polyurethane resin, a polyamide resin, a polyester resin, a polycarbonate resin, a vinyl chloride resin, an acrylonitrile-butadiene-styrene rubber, a natural rubber, a butadiene rubber, a styrene-butadiene rubber, and an acrylonitrile-butadiene rubber.

Examples of the metal include steel plates such as a stainless steel plate, a cold-rolled steel plate, and an alloyed galvanized steel plate, copper, aluminum, and magnesium alloys.

The thickness of the adherend constituting the laminate is not particularly limited. On the other hand, the thickness of the layer containing a cured product is preferably 0.01 to 2.0 mm, more preferably 0.015 to 1.5 mm, and still more preferably 0.02 to 1.2 mm, from the viewpoint of strongly bonding adherends to each other.

The method for producing a laminate is not particularly limited, and the laminate is preferably produced by the method for producing a laminate of the present invention including a bonding step of bonding a first adherend and a second adherend to each other via the cured product.

The method for bonding the first adherend and the second adherend to each other using the 1,1-dicyanoethylene-containing composition is not particularly limited; however, the first adherend and the second adherend can be bonded to each other, for example, by applying the 1,1-dicyanoethylene-containing composition to one adherend, overlaying the other adherend thereon, and curing the resultant.

The method for applying the 1,1-dicyanoethylene-containing composition to an adherend is not particularly limited, and examples thereof include a spin coating method, a spray coating method, a bar coating method, a knife coating method, a roll coating method, a roll knife coating method, a blade coating method, a die coating method, and a gravure coating method.

The amount of the 1,1-dicyanoethylene-containing composition applied to the adherend is not particularly limited, and is preferably 0.01 to 3.0 µL/mm², more preferably 0.05 to 2.5 µL/mm², and still more preferably 0.1 to 2.0 µL/mm². When the coating amount is equal to or more than the above lower limit value, it is possible to firmly bond adherends to each other. On the other hand, when the coating amount is equal to or less than the above upper limit value, it is possible to bond them with an appropriate amount.

### Examples

Hereinafter, the present invention will be described in detail by Examples, but the present invention is not limited to these Examples.

### [1,1-Dicyanoethylene-Containing Composition]

### [Component]

The components used in Examples and Comparative Examples were as follows.

### <1,1-Dicyanoethylene>

1,1-Dicyanoethylene: 1,1-dicyanoethylene produced according to the following Production Example 1 (purity: 99%)

### (Production Example 1: Production of 1,1-dicyanoethylene)

1,1-Dicyanoethylene was produced as follows.

1,1,3,3-Tetracyanopropane was synthesized in a yield of 73% from malononitrile by the production method described in J. Am. Chem. Soc., 1989, 111, 9078-9081. The obtained crystalline 1,1,3,3-tetracyanopropane and diphosphorus pentoxide were mixed and thermally decomposed at 180°C to obtain a crude product of 1,1-dicyanoethylene (yield 60%).

The crude product was purified by vacuum distillation (480 Pa) to obtain 1,1-dicyanoethylene with a purity of 99%.

### <Water>

Water: Ion-exchanged water

### <Acidic Compound (A)>

(A1): Methanesulfonic acid (manufactured by Tokyo Chemical Industry Co., Ltd. (purity > 99.0%)) (pKa = -2.6)
(A2): Sulfuric acid (manufactured by FUJIFILM Wako Pure Chemical Corporation (purity: 96 to 98%)) (pKa = -3.0)
(A3): Acetic acid (manufactured by KANTO CHEMICAL CO., INC. (purity > 99.0%)) (pKa = 4.8)
(A4): Trifluoroacetic acid (manufactured by Tokyo Chemical Industry Co., Ltd. (purity > 99.0%)) (pKa = - 0.25)
(A5): p-Toluenesulfonic acid (derived from p-toluenesulfonic acid-monohydrate) (manufactured by Tokyo Chemical Industry Co., Ltd. (purity > 98.0%)) (pKa = -2.8)

### <Polymerizable Monomer>

(B1) Ethyl 2-cyanoacrylate (manufactured by Sigma-Aldrich Co., LLC (purity > 99.9%))
(B2) Methyl methacrylate (manufactured by Tokyo Chemical Industry Co., Ltd. (purity > 99.8%))
(B3) Styrene (manufactured by Tokyo Chemical Industry Co., Ltd. (purity > 99.0%))
(B4) Vinyl acetate (manufactured by Tokyo Chemical Industry Co., Ltd. (purity > 99.0%))

### [Evaluation Methods]

The liquid 1,1-dicyanoethylene-containing compositions obtained in Examples and Comparative Examples were evaluated as samples in the following manner.

### <Storage Stability>

### (Storage Test)

A sample of 1 mL was placed in a container made of high-density polyethylene of 10 mL and tightly sealed, and three containers having a mark at a position corresponding to the sample liquid surface on the side surface of the container were prepared. The state of the sample in each container immediately before the start of the storage test was visually observed. Thereafter, the three containers were allowed to stand in an environment with a humidity of 50% at 70°C for 6 hours, at 70°C for 24 hours, and at 70°C for 48 hours, respectively. In this way, the storage test was performed.

### (Observation)

Thereafter, each container was inverted, and the state of the sample in the container was visually observed 5 seconds after the completion of the inversion.

The observation results were classified into the following three graded scores.

Score "1": 90% by volume or more of the sample in the container exceeded the mark in a side view. This meant that the sample maintained sufficient fluidity similar to that immediately before the start of the storage test (0 minutes).

Score "2": 10% by volume or more of the sample in the container exceeded the mark in a side view, but 90% by volume or more did not exceed the mark in a side view. This meant that the fluidity of the sample decreased (increased in viscosity) as compared with that immediately before the start of the storage test (0 minutes).

Score "3": Less than 10% by volume of the sample in the container exceeded the mark in a side view. This meant that polymerization of 1,1-dicyanoethylene proceeded and solidification of the sample proceeded.

### (Evaluation)

Subsequently, based on the observation results of the samples in the three containers in three grades after the storage test at 70°C, evaluation was performed according to the following criteria.
"A": The total score was 3.
"B": The total score was 4 to 7.
"C": The total score was 8 to 9.

### <Colorability>

### (Observation)

The color of the sample in the container subjected to the storage test at 70°C for 48 hours in <Storage Test> was visually observed. The observed color of the samples was classified into four stages: "transparent", "light yellow", "yellow" and "yellowish brown". Note that "yellow" means that the color was darker than "light yellow" and lighter than "yellowish brown".

### (Evaluation)

Next, based on the observation results, evaluation was performed according to the following criteria.
"A": It was "transparent".
"B": It was "light yellow".
"C": It was "yellow".
"D": It was "yellowish brown".

### <Comprehensive Evaluation of Storage Stability and Colorability>

For the evaluation results of <Storage Stability> and the evaluation results of <Colorability>, comprehensive evaluation was performed based on the following criteria.
"A": The evaluation result of the storage stability was "A" or "B", and the evaluation result of the colorability was "A".
"B": The evaluation result of the storage stability was "A" or "B", and the evaluation result of the colorability was "B" or "C".
"C": The evaluation result of the storage stability was "C", or the evaluation result of the colorability was "D".

### (Quantification of Molar Concentration Cₐ of Acidic Compound (A) in Composition)

The molar concentration of the acidic compound (A) used in Examples and Comparative Examples was quantified by ion chromatography (IC) according to the following method. In this method, even when two kinds of acidic compounds (A) are contained, they can be quantified by the same method.

First, 0.1 g of a 1,1-dicyanoethylene-containing composition was added to 99.9 g of an ion chromatography eluent (mixed aqueous solution of 0.6 mmol/L of sodium carbonate aqueous solution and 12 mmol/L of sodium hydrogen carbonate aqueous solution) to obtain a solution.

The resulting solution was stirred for 1 day. Thereafter, the solution was filtered through a filter (0.22 µm, made of PTFE). Thus, a measurement sample solution was obtained.

The measurement sample solution was measured using the following ion chromatography measurement apparatus under the following measurement conditions.

The molar concentration of the acidic compound (A) contained in the 1,1-dicyanoethylene-containing composition was quantified from a calibration curve prepared using the acidic compound (A).

### (Ion Chromatography Measurement Apparatus and Measurement Conditions)

Apparatus name: Liquid Chromatograph/10A Series, manufactured by Shimadzu Corporation
Column: Shim-pack IC-SA2 (inner diameter: 4.0 mm, length: 250 mm)
Measurement temperature: 40°C
Eluent: The eluent described above

### <Example 1>

The 1,1-dicyanoethylene 100 g (equivalent to 100 parts by mass) produced in Production Example 1 was mixed with 0.00010 parts by mass of sulfuric acid (component (A2)) under nitrogen to prepare a 1,1-dicyanoethylene-containing composition X1.

Regarding the prepared 1,1-dicyanoethylene-containing composition X1, the water content was specified by a water measurement method in accordance with JIS K0068:2001, specifically, by a coulometric titration method of the Karl Fischer titration method, and the water content was 0.055 mmol/L.

The molar concentration of the component (A2) in the 1,1-dicyanoethylene-containing composition was quantified by the above-described method (IC method) and found to be 0.010 mmol/L.

The prepared 1,1-dicyanoethylene-containing composition was sealed in a high-density polyethylene container and stored under an environment of a water concentration of 0.3 ppm.

Using the 1,1-dicyanoethylene-containing composition X1 which had been stored, the above-described evaluations of storage stability and colorability as well as comprehensive evaluation were carried out. The results are shown in Table 1.

### <Example 2>

A first 1,1-dicyanoethylene-containing composition was prepared by mixing the 1,1-dicyanoethylene 100 g (equivalent to 100 parts by mass) produced in Production Example 1 with 0.10 parts by mass of methanesulfonic acid (component (A1)) under nitrogen, and the water content and the molar concentration of the component (A1) in the first 1,1-dicyanoethylene-containing composition were specified in the same manner as in Example 1.

Next, ion-exchanged water was mixed with the first 1,1-dicyanoethylene-containing composition to obtain a second 1,1-dicyanoethylene-containing composition 2. As a result, the water content (parts by mass and molar concentration) of the second 1,1-dicyanoethylene-containing composition and the molar concentration of the component (A1) were as shown in Table 1.

The prepared second 1,1-dicyanoethylene-containing composition X2 was sealed in a high-density polyethylene container and stored under an environment of a water concentration of 0.3 ppm.

Using the second 1,1-dicyanoethylene-containing composition X2 which had been stored, the evaluations of storage stability and colorability as well as comprehensive evaluation were carried out in the same manner as in Example 1. The results are shown in Table 1.

### <Example 3, Examples 5 to 11, Examples 14 and 15, and Comparative Examples 1 to 6>

Second 1,1-dicyanoethylene-containing compositions X3, X5 to X11, X14, X15, and Y1 to Y6 were prepared in the same manner as in Example 2 so as to have the formulations shown in Tables 1 and 2. The prepared second 1,1-dicyanoethylene-containing compositions X3, X5 to X11, X14, X15, and Y1 to Y6 were stored in the same manner as in Example 2.

Using the second 1,1-dicyanoethylene-containing compositions X3, X5 to X11, X14, X15, and Y1 to Y6 which had been stored, the evaluations of storage stability and colorability as well as comprehensive evaluation were carried out in the same manner as in Example 2. The results are shown in Table 1 and Table 2.

### <Examples 4, 12, and 13>

1,1-Dicyanoethylene-containing compositions X4, X12, and X13 were prepared in the same manner as in Example 1 so as to have the formulations shown in Tables 1 and 2. The prepared 1,1-dicyanoethylene-containing compositions were stored in the same manner as in Example 1.

Using the 1,1-dicyanoethylene-containing compositions X4, X12, and X13 which had been stored, the evaluations of storage stability and colorability as well as comprehensive evaluation were carried out in the same manner as in Example 1. The results are shown in Table 1 and Table 2.

### <Example 16>

A first 1,1-dicyanoethylene-containing composition was prepared by mixing the 1,1-dicyanoethylene 100 g (equivalent to 100 parts by mass) produced in Production Example 1 with p-toluenesulfonic acid-monohydrate under nitrogen such that the amount of p-toluenesulfonic acid component (A4) was 0.001 parts by mass, and the water content and the molar concentration of the component (A4) in the first 1,1-dicyanoethylene-containing composition were specified in the same manner as in Example 1.

Next, ion-exchanged water was mixed with the first 1,1-dicyanoethylene-containing composition to obtain a second 1,1-dicyanoethylene-containing composition X16. As a result, the water content (parts by mass and molar concentration) of the second 1,1-dicyanoethylene-containing composition and the molar concentration of the component (A4) were as shown in Table 2.

The prepared second 1,1-dicyanoethylene-containing composition X16 was sealed in a high-density polyethylene container and stored under an environment of a water concentration of 0.3 ppm.

Using the second 1,1-dicyanoethylene-containing composition X16 which had been stored, the evaluations of storage stability and colorability as well as comprehensive evaluation were carried out in the same manner as in Example 1. The results are shown in Table 2.

### <Example 17 and Comparative Example 7>

1,1-Dicyanoethylene-containing compositions X17 and Y7 were prepared in the same manner as in Example 12 so as to have the formulations shown in Table 2. The prepared 1,1-dicyanoethylene-containing compositions X17 and Y7 were stored in the same manner as in Example 1.

Using the 1,1-dicyanoethylene-containing compositions X17 and Y7 which had been stored, the evaluations of storage stability and colorability as well as comprehensive evaluation were carried out in the same manner as in Example 1. The results are shown in Table 2.

### <Examples 18 to 35 and Comparative Examples 8 to 19>

Second 1,1-dicyanoethylene-containing compositions X18 to X35 and Y8 to Y19 were prepared in the same manner as in Example 2 so as to have the formulations shown in Tables 3 and 4. The prepared second 1,1-dicyanoethylene-containing compositions X18 to X35 and Y8 to Y19 were stored in the same manner as in Example 2.

Using the second 1,1-dicyanoethylene-containing compositions X18 to X35 and Y8 to Y19 which had been stored, the evaluations of storage stability and colorability as well as comprehensive evaluation were carried out in the same manner as in Example 2. The results are shown in Tables 3 and 4.

### <Reference Examples 1 to 4>

Second ethyl 2-cyanoacrylate-containing compositions Z1 to Z4 were prepared in the same manner as in Example 2 so as to have the formulations shown in Table 5. The prepared second ethyl 2-cyanoacrylate compositions Z1 to Z4 were stored in the same manner as in Example 2.

Using the second ethyl 2-cyanoacrylate-containing compositions Z1 to Z4 which had been stored, the evaluations of storage stability and colorability as well as comprehensive evaluation were carried out in the same manner as in Example 2. The results are shown in Table 5.

### [Table 1]

**Table 1**

| | | Example 1 | Example 2 | Example 3 |
|---|---|---|---|---|
| 1,1-Dicyanoethylene-containing composition | | X1 | X2 | X3 |
| 1,1-Dicyanoethylene [parts by mass] | | 100 | 100 | 100 |
| Water [parts by mass] | | 0.0001 | 0.0030 | 0.0030 |
| (A1) [parts by mass] | | - | 0.1000 | - |
| (A2) [parts by mass] | | 0.0001 | - | 0.0100 |
| (A3) [parts by mass] | | - | - | - |
| (A4) [parts by mass] | | - | - | - |
| (A5) [parts by mass] | | - | - | - |
| pKa | | -3.0 | -2.6 | -3.0 |
| C_{H2O}[mmol/L] | | 0.055 | 1.65 | 1.65 |
| Cₐ[mmol/L] | | 0.010 | 10.000 | 1.000 |
| α = pKa + Log(C_{H2O}/Cₐ) | | -2.26 | -3.38 | -2.78 |
| Storage stability | 25°C, 0 h | 1 | 1 | 1 |
| | 70°C, 6 h | 1 | 1 | 1 |
| | 70°C, 24 h | 1 | 1 | 1 |
| | 70°C, 48 h | 1 | 1 | 1 |
| | Evaluation | A | A | A |
| Colorability | 25°C, 0 h | Transparent | Transparent | Transparent |
| | 70°C, 48 h | Transparent | Transparent | Transparent |
| | Evaluation | A | A | A |
| Comprehensive evaluation | | A | A | A |

**Table 1 (continued)**

| | | Example 4 | Example 5 | Example 6 |
|---|---|---|---|---|
| 1,1-Dicyanoethylene-containing composition | | X4 | X5 | X6 |
| 1,1-Dicyanoethylene [parts by mass] | | 100 | 100 | 100 |
| Water [parts by mass] | | 0.0001 | 0.0030 | 0.0010 |
| (A1) [parts by mass] | | 0.0001 | 0.0100 | - |
| (A2) [parts by mass] | | - | - | 0.0001 |
| (A3) [parts by mass] | | - | - | - |
| (A4) [parts by mass] | | - | - | - |
| (A5) [parts by mass] | | - | - | - |
| pKa | | -2.6 | -2.6 | -3.0 |
| C_{H2O}[mmol/L] | | 0.055 | 1.65 | 0.55 |
| Cₐ[mmol/L] | | 0.010 | 1.000 | 0.010 |
| α = pKa + Log(C_{H2O}/Cₐ) | | -1.86 | -2.38 | -1.26 |
| Storage stability | 25°C, 0 h | 1 | 1 | 1 |
| | 70°C, 6 h | 1 | 1 | 1 |
| | 70°C, 24 h | 1 | 1 | 1 |
| | 70°C, 48 h | 1 | 1 | 1 |
| | Evaluation | A | A | A |
| Colorability | 25°C, 0 h | Transparent | Transparent | Transparent |
| | 70°C, 48 h | Transparent | Transparent | Transparent |
| | Evaluation | A | A | A |
| Comprehensive evaluation | | A | A | A |

### [Table 2]

**Table 2**

| | | Example 7 | Example 8 | Example 9 | Example 10 | Example 11 |
|---|---|---|---|---|---|---|
| 1,1-Dicyanoethylene-containing composition | | X7 | X8 | X9 | X10 | X11 |
| 1,1-Dicyanoethylene [parts by mass] | | 100 | 100 | 100 | 100 | 100 |
| Water [parts by mass] | | 0.0030 | 0.0010 | 0.0100 | 0.0010 | 0.0010 |
| (A1) [parts by mass] | | 0.0010 | 0.0001 | - | 0.1000 | 0.0010 |
| (A2) [parts by mass] | | - | - | 0.0001 | - | - |
| (A3) [parts by mass] | | - | - | - | - | - |
| (A4) [parts by mass] | | - | - | - | - | - |
| (A5) [parts by mass] | | - | - | - | - | - |
| pKa | | -2.6 | -2.6 | -3.0 | -2.6 | -2.6 |
| C_{H2O}[mmol/L] | | 1.65 | 0.55 | 5.50 | 0.55 | 0.55 |
| Cₐ[mmol/L] | | 0.10 | 0.010 | 0.010 | 10.0 | 0.10 |
| α = pKa + Log(C_{H2O}/Cₐ) | | -1.38 | -0.86 | -0.26 | -3.86 | -1.86 |
| Storage stability | 25°C, 0 h | 1 | 1 | 1 | 1 | 1 |
| | 70°C, 6 h | 1 | 1 | 1 | 1 | 1 |
| | 70°C, 24 h | 1 | 2 | 2 | 2 | 1 |
| | 70°C, 48 h | 2 | 3 | 3 | 3 | 1 |
| | Evaluation | B | B | B | B | A |
| Colorability | 25°C, 0 h | Transparent | Transparent | Transparent | Transparent | Transparent |
| | 70°C, 48 h | Transparent | Transparent | Transparent | Light yellow | Transparent |
| | Evaluation | A | A | A | B | A |
| Comprehensive evaluation | | A | A | A | B | A |

**Table 2 (continued)**

| | | Example 12 | Example 13 | Example 14 | Example 15 |
|---|---|---|---|---|---|
| 1,1-Dicyanoethylene-containing composition | | X12 | X13 | X14 | X15 |
| 1,1-Dicyanoethylene [parts by mass] | | 100 | 100 | 100 | 100 |
| Water [parts by mass] | | 0.0001 | 0.0001 | 1.0000 | 0.1000 |
| (A1) [parts by mass] | | - | - | 10.0000 | 0.5000 |
| (A2) [parts by mass] | | - | - | - | - |
| (A3) [parts by mass] | | - | - | - | - |
| (A4) [parts by mass] | | 0.0001 | 0.0010 | - | - |
| (A5) [parts by mass] | | - | - | - | - |
| pKa | | -0.25 | -0.25 | -2.6 | -2.6 |
| C_{H2O}[mmol/L] | | 0.055 | 0.055 | 496 | 54.8729 |
| Cₐ[mmol/L] | | 0.087 | 0.870 | 1394.5 | 51.390 |
| α = pKa + Log(C_{H2O}/Cₐ) | | -0.45 | -1.45 | -3.05 | -2.57 |
| Storage stability | 25°C, 0 h | 1 | 1 | 1 | 1 |
| | 70°C, 6 h | 1 | 1 | 1 | 1 |
| | 70°C, 24 h | 2 | 2 | 2 | 1 |
| | 70°C, 48 h | 3 | 3 | 3 | 2 |
| | Evaluation | B | B | B | B |
| Colorability | 25°C, 0 h | Transparent | Transparent | Transparent | Transparent |
| | 70°C, 48 h | Transparent | Light yellow | Yellowish brown | Transparent |
| | Evaluation | A | B | B | A |
| Comprehensive evaluation | | A | B | B | A |

**Table 2 (continued)**

| | | Example 16 | Example 17 | Comparative Example 1 | Comparative Example 2 |
|---|---|---|---|---|---|
| 1,1-Dicyanoethylene-containing composition | | X16 | X17 | Y1 | Y2 |
| 1,1-Dicyanoethylene [parts by mass] | | 100 | 100 | 100 | 100 |
| Water [parts by mass] | | 0.0010 | 0.0010 | 0.0030 | 0.0010 |
| (A1) [parts by mass] | | - | - | - | 1.0000 |
| (A2) [parts by mass] | | - | - | 0.3000 | - |
| (A3) [parts by mass] | | - | - | - | - |
| (A4) [parts by mass] | | - | - | - | - |
| (A5) [parts by mass] | | 0.0010 | 0.0100 | - | - |
| pKa | | -2.8 | -2.8 | -3.0 | -2.6 |
| C_{H2O}[mmol/L] | | 0.55 | 0.55 | 1.65 | 0.55 |
| Cₐ[mmol/L] | | 0.0058 | 0.058 | 30.0 | 100.0 |
| α = pKa + Log(C_{H2O}/Cₐ) | | -0.82 | -1.82 | -4.26 | -4.86 |
| Storage stability | 25°C, 0 h | 1 | 1 | 1 | 1 |
| | 70°C, 6 h | 1 | 1 | 3 | 3 |
| | 70°C, 24 h | 2 | 1 | 3 | 3 |
| | 70°C, 48 h | 3 | 1 | 3 | 3 |
| | Evaluation | B | A | C | C |
| Colorability | 25°C, 0 h | Transparent | Transparent | Transparent | Transparent |
| | 70°C, 48 h | Transparent | Transparent | Yellowish brown | Yellowish brown |
| | Evaluation | A | A | D | D |
| Comprehensive evaluation | | A | A | C | C |

**Table 2 (continued)**

| | | Comparative Example 3 | Comparative Example 5 | Comparative Example 6 | Comparative Example 7 |
|---|---|---|---|---|---|
| 1,1-Dicyanoethylene-containing composition | | Y3 | Y4 | Y5 | Y7 |
| 1,1-Dicyanoethylene [parts by mass] | | 100 | 100 | 100 | 100 |
| Water [parts by mass] | | 0.0100 | 0.0030 | 0.0100 | 0.0100 |
| (A1) [parts by mass] | | 0.0001 | - | - | - |
| (A2) [parts by mass] | | - | - | - | - |
| (A3) [parts by mass] | | - | 0.0100 | - | - |
| (A4) [parts by mass] | | - | - | 0.0001 | - |
| (A5) [parts by mass] | | - | - | - | 0.0001 |
| pKa | | -2.6 | 4.8 | -0.25 | -2.8 |
| C_{H2O}[mmol/L] | | 5.5 | 1.65 | 5.5 | 5.5 |
| Cₐ[mmol/L] | | 0.010 | 1.0 | 0.0087 | 0.0058 |
| α = pKa + Log(C_{H2O}/Cₐ) | | 0.14 | 5.02 | 2.55 | 0.18 |
| Storage stability | 25°C, 0 h | 1 | 1 | 1 | 1 |
| | 70°C, 6 h | 3 | 3 | 3 | 3 |
| | 70°C, 24 h | 3 | 3 | 3 | 3 |
| | 70°C, 48 h | 3 | 3 | 3 | 3 |
| | Evaluation | C | C | C | C |
| Colorability | 25°C, 0 h | Transparent | Transparent | Transparent | Transparent |
| | 70°C, 48 h | Transparent | Transparent | Transparent | Transparent |
| | Evaluation | A | A | A | A |
| Comprehensive evaluation | | C | C | C | C |

### [Table 3]

**Table 3**

| | | Example 18 | Example 19 | Example 20 | Example 21 | Example 22 |
|---|---|---|---|---|---|---|
| 1,1-Dicyanoethylene-containing composition | | X18 | X19 | X20 | X21 | X22 |
| 1,1-Dicyanoethylene [parts by mass] | | 99 | 95 | 80 | 50 | 20 |
| (B1) [parts by mass] | | 1 | 5 | 20 | 50 | 80 |
| (B2) [parts by mass] | | - | - | - | - | - |
| (B3) [parts by mass] | | - | - | - | - | - |
| (B4) [parts by mass] | | - | - | - | - | - |
| Water [parts by mass] | | 0.0010 | 0.0010 | 0.0010 | 0.0010 | 0.0010 |
| (A1) [parts by mass] | | 0.0010 | 0.0010 | 0.0010 | 0.0010 | 0.0010 |
| (A2) [parts by mass] | | - | - | - | - | - |
| pKa | | -2.6 | -2.6 | -2.6 | -2.6 | -2.6 |
| C_{H2O}[mmol/L] | | 0.0055 | 0.0055 | 0.0056 | 0.0057 | 0.0058 |
| Cₐ[mmol/L] | | 0.0010 | 0.0010 | 0.0010 | 0.0011 | 0.0011 |
| α = pKa + Log(C_{H2O}/Cₐ) | | -1.87 | -1.87 | -1.87 | -1.87 | -1.87 |
| Storage stability | 25°C, 0 h | 1 | 1 | 1 | 1 | 1 |
| | 70°C, 6 h | 1 | 1 | 1 | 1 | 1 |
| | 70°C, 24 h | 1 | 1 | 1 | 1 | 1 |
| | 70°C, 48 h | 1 | 1 | 1 | 1 | 1 |
| | Evaluation | A | A | A | A | A |
| Colorability | 25°C, 0 h | Transparent | Transparent | Transparent | Transparent | Transparent |
| | 70°C, 48 h | Transparent | Transparent | Transparent | Transparent | Transparent |
| | Evaluation | A | A | A | A | A |
| Comprehensive evaluation | | A | A | A | A | A |

**Table 3 (continued)**

| | | Example 23 | Example 24 | Example 25 | Example 26 | Example 27 |
|---|---|---|---|---|---|---|
| 1,1-Dicyanoethylene-containing composition | | X23 | X24 | X25 | X26 | X27 |
| 1,1-Dicyanoethylene [parts by mass] | | 10 | 5 | 1 | 99 | 95 |
| (B1) [parts by mass] | | 90 | 95 | 99 | 1 | 5 |
| (B2) [parts by mass] | | - | - | - | - | - |
| (B3) [parts by mass] | | - | - | - | - | - |
| (B4) [parts by mass] | | - | - | - | - | - |
| Water [parts by mass] | | 0.0010 | 0.0010 | 0.0010 | 0.0010 | 0.0010 |
| (A1) [parts by mass] | | 0.0010 | 0.0010 | 0.0010 | - | - |
| (A2) [parts by mass] | | - | - | - | 0.0001 | 0.0001 |
| pKa | | -2.6 | -2.6 | -2.6 | -3.0 | -3.0 |
| C_{H2O}[mmol/L] | | 0.0059 | 0.0059 | 0.0059 | 0.0055 | 0.0055 |
| Cₐ[mmol/L] | | 0.0011 | 0.0011 | 0.0011 | 0.0001 | 0.0001 |
| α = pKa + Log(C_{H2O}/Cₐ) | | -1.87 | -1.87 | -1.87 | -1.26 | -1.26 |
| Storage stability | 25°C, 0 h | 1 | 1 | 1 | 1 | 1 |
| | 70°C, 6 h | 1 | 1 | 1 | 1 | 1 |
| | 70°C, 24 h | 1 | 1 | 1 | 1 | 1 |
| | 70°C, 48 h | 1 | 1 | 1 | 1 | 1 |
| | Evaluation | A | A | A | A | A |
| Colorability | 25°C, 0 h | Transparent | Transparent | Transparent | Transparent | Transparent |
| | 70°C, 48 h | Transparent | Transparent | Transparent | Transparent | Transparent |
| | Evaluation | A | A | A | A | A |
| Comprehensive evaluation | | A | A | A | A | A |

**Table 3 (continued)**

| | | Example 28 | Example 29 | Example 30 | Example 31 |
|---|---|---|---|---|---|
| 1,1-Dicyanoethylene-containing composition | | X28 | X29 | X30 | X31 |
| 1,1-Dicyanoethylene [parts by mass] | | 5 | 95 | 95 | 5 |
| (B1) [parts by mass] | | 99 | - | - | - |
| (B2) [parts by mass] | | - | - | - | 95 |
| (B3) [parts by mass] | | - | 5 | - | - |
| (B4) [parts by mass] | | - | - | 5 | - |
| Water [parts by mass] | | 0.0010 | 0.0010 | 0.0010 | 0.0010 |
| (A1) [parts by mass] | | - | 0.0010 | 0.0010 | 0.0010 |
| (A2) [parts by mass] | | 0.0001 | - | - | - |
| pKa | | -3.0 | -2.6 | -2.6 | -2.6 |
| C_{H2O}[mmol/L] | | 0.0061 | 0.0055 | 0.0055 | 0.0052 |
| Cₐ[mmol/L] | | 0.0001 | 0.0010 | 0.0010 | 0.0010 |
| α = pKa + Log(C_{H2O}/Cₐ) | | -1.26 | -1.87 | -1.87 | -1.87 |
| Storage stability | 25°C, 0 h | 1 | 1 | 1 | 1 |
| | 70°C, 6 h | 1 | 1 | 1 | 1 |
| | 70°C, 24 h | 1 | 1 | 1 | 1 |
| | 70°C, 48 h | 1 | 1 | 1 | 1 |
| | Evaluation | A | A | A | A |
| Colorability | 25°C, 0 h | Transparent | Transparent | Transparent | Transparent |
| | 70°C, 48 h | Transparent | Transparent | Transparent | Transparent |
| | Evaluation | A | A | A | A |
| Comprehensive evaluation | | A | A | A | A |

**Table 3 (continued)**

| | | Example 32 | Example 33 | Example 34 | Example 35 |
|---|---|---|---|---|---|
| 1,1-Dicyanoethylene-containing composition | | X32 | X33 | X34 | X35 |
| 1,1-Dicyanoethylene [parts by mass] | | 5 | 1 | 95 | 5 |
| (B1) [parts by mass] | | - | 99 | - | - |
| (B2) [parts by mass] | | - | - | 5 | - |
| (B3) [parts by mass] | | 95 | - | - | - |
| (B4) [parts by mass] | | - | - | - | 95 |
| Water [parts by mass] | | 0.0010 | 0.0010 | 0.0010 | 0.0010 |
| (A1) [parts by mass] | | 0.0010 | - | 0.0010 | 0.0010 |
| (A2) [parts by mass] | | - | 0.0001 | - | - |
| pKa | | -2.6 | -3.0 | -2.6 | -2.6 |
| C_{H2O}[mmol/L] | | 0.0051 | 0.0059 | 0.0055 | 0.0052 |
| Cₐ[mmol/L] | | 0.0009 | 0.0001 | 0.0010 | 0.0010 |
| α = pKa + Log(C_{H2O}/Cₐ) | | -1.87 | -1.26 | -1.87 | -1.87 |
| Storage stability | 25°C, 0 h | 1 | 1 | 1 | 1 |
| | 70°C, 6 h | 1 | 1 | 1 | 1 |
| | 70°C, 24 h | 1 | 1 | 1 | 1 |
| | 70°C, 48 h | 1 | 1 | 1 | 1 |
| | Evaluation | A | A | A | A |
| Colorability | 25°C, 0 h | Transparent | Transparent | Transparent | Transparent |
| | 70°C, 48 h | Transparent | Transparent | Transparent | Transparent |
| | Evaluation | A | A | A | A |
| Comprehensive evaluation | | A | A | A | A |

### [Table 4]

**Table 4**

| | | Comparative Example 8 | Comparative Example 9 | Comparative Example 10 | Comparative Example 11 |
|---|---|---|---|---|---|
| 1,1-Dicyanoethylene-containing composition | | Y8 | Y9 | Y10 | Y11 |
| 1,1-Dicyanoethylene [parts by mass] | | 99 | 95 | 80 | 50 |
| (B1) [parts by mass] | | 1 | 5 | 20 | 50 |
| (B2) [parts by mass] | | - | - | - | - |
| (B3) [parts by mass] | | - | - | - | - |
| (B4) [parts by mass] | | - | - | - | - |
| Water [parts by mass] | | 0.0100 | 0.0100 | 0.0100 | 0.0100 |
| (A1) [parts by mass] | | 0.0001 | 0.0001 | 0.0001 | 0.0001 |
| (A2) [parts by mass] | | - | - | - | - |
| pKa | | -2.6 | -2.6 | -2.6 | -2.6 |
| C_{H2O}[mmol/L] | | 0.055 | 0.055 | 0.056 | 0.057 |
| Cₐ[mmol/L] | | 0.00010 | 0.00010 | 0.00010 | 0.00011 |
| α = pKa + Log(C_{H2O}/Cₐ) | | 0.13 | 0.13 | 0.13 | 0.13 |
| Storage stability | 25°C, 0 h | 1 | 1 | 1 | 1 |
| | 70°C, 6 h | 3 | 3 | 3 | 3 |
| | 70°C, 24 h | 3 | 3 | 3 | 3 |
| | 70°C, 48 h | 3 | 3 | 3 | 3 |
| | Evaluation | C | C | C | C |
| Colorability | 25°C, 0 h | Transparent | Transparent | Transparent | Transparent |
| | 70°C, 48 h | Transparent | Transparent | Transparent | Transparent |
| | Evaluation | A | A | A | A |
| Comprehensive evaluation | | C | C | C | C |

**Table 4 (continued)**

| | | Comparative Example 12 | Comparative Example 13 | Comparative Example 14 | Comparative Example 15 |
|---|---|---|---|---|---|
| 1,1-Dicyanoethylene-containing composition | | Y12 | Y13 | Y14 | Y15 |
| 1,1-Dicyanoethylene [parts by mass] | | 20 | 50 | 5 | 1 |
| (B1) [parts by mass] | | 80 | 50 | 95 | 99 |
| (B2) [parts by mass] | | - | - | - | - |
| (B3) [parts by mass] | | - | - | - | - |
| (B4) [parts by mass] | | - | - | - | - |
| Water [parts by mass] | | 0.0100 | 0.0100 | 0.0100 | 0.0100 |
| (A1) [parts by mass] | | 0.0001 | 0.0001 | 0.0001 | 0.0001 |
| (A2) [parts by mass] | | - | - | - | - |
| pKa | | -2.6 | -2.6 | -2.6 | -2.6 |
| C_{H2O}[mmol/L] | | 0.058 | 0.057 | 0.059 | 0.059 |
| Cₐ[mmol/L] | | 0.00011 | 0.00011 | 0.00011 | 0.00011 |
| α = pKa + Log(C_{H2O}/Cₐ) | | 0.13 | 0.13 | 0.13 | 0.13 |
| Storage stability | 25°C, 0 h | 1 | 1 | 1 | 1 |
| | 70°C, 6 h | 3 | 3 | 3 | 3 |
| | 70°C, 24 h | 3 | 3 | 3 | 3 |
| | 70°C, 48 h | 3 | 3 | 3 | 3 |
| | Evaluation | C | C | C | C |
| Colorability | 25°C, 0 h | Transparent | Transparent | Transparent | Transparent |
| | 70°C, 48 h | Transparent | Transparent | Transparent | Transparent |
| | Evaluation | A | A | A | A |
| Comprehensive evaluation | | C | C | C | C |

**Table 4 (continued)**

| | | Comparative Example 16 | Comparative Example 17 | Comparative Example 18 | Comparative Example 19 |
|---|---|---|---|---|---|
| 1,1-Dicyanoethylene-containing composition | | Y16 | Y17 | Y18 | Y19 |
| 1,1-Dicyanoethylene [parts by mass] | | 95 | 5 | 95 | 5 |
| (B1) [parts by mass] | | 5 | 95 | 5 | 95 |
| (B2) [parts by mass] | | - | - | - | - |
| (B3) [parts by mass] | | - | - | - | - |
| (B4) [parts by mass] | | - | - | - | - |
| Water [parts by mass] | | 0.0010 | 0.0010 | 0.0030 | 0.0030 |
| (A1) [parts by mass] | | 1.0000 | 1.0000 | - | - |
| (A2) [parts by mass] | | - | - | 0.30 | 0.30 |
| pKa | | -2.6 | -2.6 | -3.0 | -3.0 |
| C_{H2O}[mmol/L] | | 0.553 | 0.587 | 1.659 | 1.761 |
| Cₐ[mmol/L] | | 100.0 | 100.0 | 30.0 | 30.0 |
| α = pKa + Log(C_{H2O}/Cₐ) | | -4.86 | -4.83 | -4.26 | -4.23 |
| Storage stability | 25°C, 0 h | 1 | 1 | 1 | 1 |
| | 70°C, 6 h | 3 | 3 | 3 | 3 |
| | 70°C, 24 h | 3 | 3 | 3 | 3 |
| | 70°C, 48 h | 3 | 3 | 3 | 3 |
| | Evaluation | C | C | C | C |
| Colorability | 25°C, 0 h | Transparent | Transparent | Transparent | Transparent |
| | 70°C, 48 h | Yellowish brown | Yellowish brown | Yellowish brown | Yellowish brown |
| | Evaluation | D | D | D | D |
| Comprehensive evaluation | | C | C | C | C |

### [Table 5]

**Table 5**

| | | Reference Example 1 | Reference Example 2 | Reference Example 3 | Reference Example 4 |
|---|---|---|---|---|---|
| Ethyl 2-cyanoacrylate-containing composition | | Z1 | Z2 | Z3 | Z4 |
| 1,1-Dicyanoethylene [parts by mass] | | | | | |
| (B1) [parts by mass] | | 100 | 100 | 100 | 100 |
| (B2) [parts by mass] | | - | - | - | - |
| (B3) [parts by mass] | | - | - | - | - |
| (B4) [parts by mass] | | - | - | - | - |
| Water [parts by mass] | | 0.0030 | 0.0010 | 0.010 | 0.0010 |
| (A1) [parts by mass] | | - | 1.0 | 0.00010 | 0.10 |
| (A2) [parts by mass] | | 0.30 | - | - | - |
| pKa | | -2.6 | -2.6 | -2.6 | -2.6 |
| C_{H2O}[mmol/L] | | 0.018 | 0.0059 | 0.0589 | 0.0059 |
| Cₐ[mmol/L] | | 0.32 | 1.10 | 0.00011 | 0.11 |
| α = pKa + Log(C_{H2O}/Cₐ) | | -3.86 | -4.87 | 0.13 | -3.87 |
| Storage stability | 25°C, 0 h | 1 | 1 | 1 | 1 |
| | 70°C, 6 h | 1 | 1 | 1 | 1 |
| | 70°C, 24 h | 1 | 1 | 2 | 1 |
| | 70°C, 48 h | 1 | 1 | 3 | 1 |
| | Evaluation | A | A | B | A |
| Colorability | 25°C, 0 h | Transparent | Transparent | Transparent | Transparent |
| | 70°C, 48 h | Transparent | Transparent | Transparent | Transparent |
| | Evaluation | A | A | A | A |
| Comprehensive evaluation | | A | A | A | A |

From Tables 1 and 2, it was found that the 1,1-dicyanoethylene-containing compositions according to Examples 1 to 17 were excellent in storage stability as compared with the 1,1-dicyanoethylene-containing compositions according to Comparative Examples 1 to 7. In addition, from Comparative Examples 3, 4, 5, and 6, it was found that it is difficult to obtain a 1,1-dicyanoethylene-containing composition having excellent storage stability when α is more than 0. Furthermore, it was found that in a case when the molar concentration of the acidic compound was higher than the molar concentration of water as in Examples 10, 13, and 14, the evaluation of the colorability tended to be slightly inferior to that of Examples 1 to 9.

From Tables 3 and 4, it was found that the 1,1-dicyanoethylene-containing compositions according to Examples 18 to 35 were excellent in storage stability and colorability as compared with the 1,1-dicyanoethylene-containing compositions according to Comparative Examples 8 to 19 even when the polymerizable monomer (B) was contained. Furthermore, as shown in Reference Examples 1 to 4 (Table 5), the compositions not containing 1,1-dicyanoethylene were more excellent in storage stability and colorability than the compositions containing 1,1-dicyanoethylene as in Comparative Examples 1 to 3 and Example 10, and thus it was found that these are properties specific to 1,1-dicyanoethylene-containing compositions.

### [Laminate]

### [Adherend]

- Steel plate (manufactured by Standard Test Piece Co., Ltd., height 25 mm, width 100 mm, thickness 1.6 mm)
- Aluminum plate (manufactured by Standard Test Piece Co., Ltd., height 25 mm, width 100 mm, thickness 1.6 mm)
- Polyvinyl chloride (PVC) plate (manufactured by Nippon Testpanel Co., Ltd., height 25 mm, width 100 mm, thickness 2.0 mm)

### [Measurement Method]

The tensile shear adhesive strength was measured as follows using the laminates obtained in Examples and Comparative Examples as samples.

### <Evaluation of Tensile Shear Adhesive Strength>

The tensile shear adhesive strength of the laminate was measured at 25°C and 25% RH using a universal material tester Model 5969 (manufactured by Instron Corporation) at a tensile speed of 20 mm/min.

### <Examples 36 to 44 and Comparative Examples 20 to 22>

A 1,1-dicyanoethylene-containing composition (100 µL) shown in Table 6 was applied to an area of 12.5 mm × 25 mm of an adherend (1) shown in Table 6, and an adherend (2) shown in Table 6 was stacked on the applied surface. Thereafter, the 1,1-dicyanoethylene-containing composition was allowed to stand for 1 day in an environment at a room temperature of 23°C and a humidity of 50% to cure the 1,1-dicyanoethylene-containing composition, thereby bonding the adherends (1) and (2) to obtain a laminate.

Using the obtained laminate, the tensile shear adhesive strength was measured. The results are shown in Table 6.

### [Table 6]

**Table 6**

| | Example 36 | Example 37 | Example 38 | Example 39 | Example 40 | Example 41 |
|---|---|---|---|---|---|---|
| 1,1-Dicyanoethylene-containing composition | X1 | X1 | X1 | X18 | X19 | X20 |
| Adherend (1) | Steel plate | Aluminum | PVC | Steel plate | Steel plate | Steel plate |
| Adherend (2) | Steel plate | Steel plate | PVC | Steel plate | Steel plate | Steel plate |
| Tensile shear adhesive strength [MPa] | 12.0 | 12.5 | Adherend failure | 12.3 | 12.4 | 12.5 |

**Table 6 (continued)**

| | Example 42 | Example 43 | Example 44 | Comparative Example 20 | Comparative Example 21 | Comparative Example 22 |
|---|---|---|---|---|---|---|
| 1,1-Dicyanoethylene-containing composition | X21 | X22 | X23 | Y2 | Y3 | Y4 |
| Adherend (1) | Steel plate | Steel plate | Steel plate | Steel plate | Steel plate | Steel plate |
| Adherend (2) | Steel plate | Steel plate | Steel plate | Steel plate | Steel plate | Steel plate |
| Tensile shear adhesive strength [MPa] | 12.5 | 13.2 | 14.3 | 8.5 | 6.0 | 2.0 |

From Table 6, it was found that the laminates obtained by curing the 1,1-dicyanoethylene-containing compositions according to Examples 36 to 44 were excellent in tensile shear adhesive strength as compared with the laminates obtained by curing the 1,1-dicyanoethylene-containing compositions according to Comparative Examples 20 to 22.

According to the present Examples, since a 1,1-dicyanoethylene-containing composition having excellent storage stability and a method for producing the same can be provided, it has been found that even in a cured product and a laminate thereof, it can be expected to be able to suppress a decrease in physical properties due to the 1,1-dicyanoethylene-containing composition impairing storage stability.

## Claims

1. A 1,1-dicyanoethylene-containing composition comprising 1,1-dicyanoethylene, water, and an acidic compound (A), wherein a content of the acidic compound (A) and a content of the water in the composition satisfy the following relational expression (Ia): $- 4.00 \leq α \leq 0$ in the relational expression (Ia), α is represented by the following formula (Ib): $α = pKa + Log \left({C}_{H 2 O}/{C}_{a}\right)$ in the formula (Ib), pKa is a logarithmic acid dissociation constant of the acidic compound (A); C_{H2O} is a molar concentration of the water in the composition; and Cₐ is a molar concentration of the acidic compound (A) in the composition.

2. The 1,1-dicyanoethylene-containing composition according to claim 1, wherein the acidic compound (A) is selected from acidic compounds having a logarithmic acid dissociation constant pKa of -1.7 or less.

3. The 1,1-dicyanoethylene-containing composition according to claim 1, wherein the acidic compound (A) is at least one selected from methanesulfonic acid, sulfuric acid, p-toluenesulfonic acid, and hydrochloric acid.

4. The 1,1-dicyanoethylene-containing composition according to claim 1, further comprising a polymerizable monomer (B).

5. The 1,1-dicyanoethylene-containing composition according to claim 4, wherein the polymerizable monomer (B) is one or more selected from the group consisting of ethylene, propylene, butadiene, isobutylene, isoprene, 1-hexene, 1-octene, vinyl acetate, vinyl propionate, vinyl butyrate, styrene, α-methylstyrene, p-methylstyrene, acrylic acid, methacrylic acid, alkyl acrylate, alkyl methacrylate, acrylonitrile, vinyl chloride, vinylidene chloride, vinylidene fluoride, 2-cyanoacrylic acid alkyl ester, 2-cyanopentadienoic acid alkyl ester, and methylidenemalonic acid dialkyl ester.

6. The 1,1-dicyanoethylene-containing composition according to claim 5, wherein the polymerizable monomer (B) comprises 2-cyanoacrylic acid alkyl ester.

7. The 1,1-dicyanoethylene-containing composition according to claim 6, wherein the 2-cyanoacrylic acid alkyl ester is ethyl 2-cyanoacrylate.

8. The 1,1-dicyanoethylene-containing composition according to claim 1, wherein the molar concentration of the acidic compound (A) in the composition is 1,000 mmol/L or less.

9. A cured product obtained by reacting the 1,1-dicyanoethylene-containing composition according to any one of claims 1 to 8.

10. A laminate comprising: a cured product obtained by reacting the 1,1-dicyanoethylene-containing composition according to any one of claims 1 to 8; and an adherend bonded to the cured product.

11. A method for producing a 1,1-dicyanoethylene-containing composition, the method comprising a step of blending an acidic compound (A) such that a content of the acidic compound (A) in the composition and a content of water in the composition satisfy the following relational expression (Ia):$- 4.00 \leq α \leq 0$
in the relational expression (Ia), α is represented by the following formula (Ib):$α = pKa + Log \left({C}_{H 2 O}/{C}_{a}\right)$ in the formula (Ib), pKa is a logarithmic acid dissociation constant of the acidic compound (A); C_{H2O} is a molar concentration of the water in the composition; and Cₐ is a molar concentration of the acidic compound (A) in the composition.
